# FASCICULE DE BREVET EUROPEEN

(11) **EP 0 737 509 B1**
(45) Date de publication et mention de la délivrance du brevet: **05.07.2000**
(21) Numéro de dépôt: 96400739.7
(22) Date de dépôt: 05.04.1996
(51) Int. Cl.: B01J 2/04, A61K 9/16

(54) **Perles cristallisées d'un produit présentant le phénomène de surfusion et leur procédé d'obtention**
Kristallierte Perlen aus einem Produkt das unterkühlt werden kann und Verfahren zu ihrer Herstellung
Crystallized beads of a product which can be supercooled and process to prepare them

(30) Priorité: 10.04.1995 FR 9504260
(43) Date de publication de la demande: 16.10.1996
(73) Titulaire: RHODIA CHIMIE, 92408 Courbevoie Cédex (FR)
(72) Inventeur: Le Thiesse, Jean-Claude, 42100 Saint-Etienne (FR); Statiotis, Eraclis, 38280 Vilette d'Anthon (FR)
(74) Mandataire: Dutruc-Rosset, Marie-Claude

(56) Documents cités:
- EP-A- 0 438 359
- WO-A-81/02890
- WO-A-91/19484
- US-A- 3 450 804
- DATABASE WPI Derwent Publications Ltd., London, GB; AN 95-091129 XP002007370 & BR-A-9 401 431 (CIBA GEIGY AG) , 13 Décembre 1994

## Description

La présente invention a pour objet une nouvelle présentation d'un produit présentant le phénomène de surfusion. Plus précisément, l'invention a pour objet des perles cristallisées d'un produit présentant une forte surfusion. L'invention se rapporte aussi à un procédé de préparation desdites perles.

Dans de multiples domaines d'application, on requiert que les produits mis en oeuvre se trouvent sous une forme telle qu'elle doit satisfaire à de nombreuses exigences :
- le produit doit être aisément manipulable,
- la répartition granulométrique du produit doit exclure des particules fines qui génèrent des poussières, particules inférieures à 100 µm environ,
- le produit doit présenter de bonnes propriétés d'écoulement et ne pas motter dans les installations de stockage temporaire, du type trémie ou lors d'une utilisation dans des processus de transformation ultérieure, ou au cours d'un stockage prolongé dans les emballages usuels.

Ces exigences peuvent être plus ou moins satisfaites en mettant à disposition le produit sous la forme d'une poudre à grosse granulométrie.

On peut envisager d'obtenir une telle granulométrie par une opération de cristallisation en solution. Toutefois, une telle technique possède ses limites car il n'est pas toujours facile de contrôler la croissance cristalline et, dans le cas d'anisotropie, les cristaux obtenus sont fragiles et ne répondent pas aux impératifs en matière de propriétés mécaniques.

Il est également possible de faire appel à des techniques de mise en forme d'une poudre comme la granulation mais, en général, ces techniques nécessitent l'utilisation d'un additif comme liant. On peut également mettre en oeuvre la technique de "prilling" qui consiste à fondre le produit, à le fractionner en gouttelettes qui sont solidifiées par refroidissement.

Le problème qui se pose alors est que de nombreux produits, notamment les matières actives dans le domaine pharmaceutique ou agrochimique, présentent un phénomène de surfusion, c'est-à-dire que lorsque le produit est fondu et qu'il est refroidi au-dessous de son point de fusion, il ne cristallise pas et reste à l'état liquide. Il s'ensuit que lorsqu'un produit présente le phénomène de surfusion et c'est le cas notamment du gaïacolate de glycéryle, il existe de réelles difficultés à mettre en oeuvre une technique de prilling.

EP 0 438 359 A1 décrit un procédé de préparation de perles à base d'un principe actif pharmaceutique présentant un point de cristallisation non défini caractérisé en ce qu'on mélange sous forme fondue ledit principe actif avec un excipient pharmaceutique favorisant la solidification du principe actif, on force le passage de la masse fondue à travers une buse qui subit une vibration, on laisse tomber dans une tour à contre courant avec un gaz les pertes formées, on rassemble les perles formées dans le bas de la tour.

L'objet précisément de la présente invention est de fournir un procédé nouveau permettant de mettre en forme un produit présentant de la surfusion.

Un autre objet de l'invention est qu'il conduit à un produit satisfaisant toutes les exigences précitées.

Enfin, un autre objet est une nouvelle forme ou présentation d'un produit présentant un phénomène de surfusion, sans qu'il y soit inclus un additif de cristallisation.

La présente invention a pour objet des perles cristallisées d'un produit présentant une surfusion telle que l'on mette en évidence une transition vitreuse et ne comprenant pas d'additifs de cristallisation.

Dans l'exposé de la présente invention, on entend par "perles", des particules solides à forte sphéricité.

La caractéristique du procédé de l'invention, en vue de préparer des perles cristallisées d'un produit présentant le phénomène de surfusion consiste :
- à faire fondre si nécessaire ledit produit,
- à fragmenter la masse fondue en gouttelettes,
- à refroidir les gouttelettes à une température inférieure à la température de transition vitreuse du produit afin que ces gouttelettes se durcissent par vitrification,
- à maintenir les perles ainsi vitrifiées à une température inférieure à la température de transition vitreuse et à les mettre en contact avec des germes de cristallisation,
- à faire cristalliser les perles en élevant progressivement la température au-dessus de la température de transition vitreuse,
- à récupérer les perles cristallisées.

Une variante préférée du procédé de l'invention consiste à faire fondre si nécessaire le produit à mettre en forme, puis à faire passer la masse fondue dans une buse de façon à former des gouttelettes, à solidifier ces dernières en les laissant tomber dans une tour à contre-courant d'un gaz froid, puis à récupérer les perles vitrifiées qui sont ensuite transformées en perles cristallisées selon les étapes précédemment décrites.

Le procédé de l'invention est parfaitement bien adapté pour la préparation de perles d'un produit présentant une forte surfusion.

On rappellera que par "surfusion", on définit l'état d'un corps qui reste liquide en dessous de sa température de fusion.

Le procédé de l'invention convient tout à fait bien pour la mise en forme notamment de produits organiques utilisés dans le domaine pharmaceutique et agrochimique.

Comme exemples spécifiques, on peut citer le gaïacolate de glycéryle, les dérivés de l'acide propionique et plus particulièrement l'acide 2-(3-benzoylphényl)propionique (Kétoprofène®), l'acide 2-(4-isobutylphényl)propionique (Ibuprofène®), le dicarbonate de 2-méthyl-2-propyl-1,3-propanediol ou méprobamate.

Les perles obtenues selon l'invention présentent des caractéristiques physico-chimiques qui leur sont propres.

Les perles obtenues présentent une taille de particules essentiellement sous forme sphérique, ayant un diamètre qui peut être choisi, grâce au procédé de l'invention, dans une large gamme. Ainsi, la taille des particules exprimée par le diamètre médian (d₅₀) peut s'échelonner entre 100 µm et 3 000 µm mais se situe, de préférence, entre 500 µm et 2 000 µm.

On définit le diamètre médian comme étant tel que 50 % en poids des particules ont un diamètre supérieur ou inférieur au diamètre médian.

Précisons que la détermination des tailles se fait par passage sur des tamis métalliques.

Selon le produit de départ et son domaine d'application, la granulométrie choisie peut être plus ou moins grosse. Ainsi, dans le cas du gaïacolate de glycéryle qui constitue une application préférentielle du procédé de l'invention, le diamètre médian se situe, de préférence entre 500 µm et 1 000 µm.

La figure 1 représente une photographie prise au microscope optique (G = 18) qui montre la morphologie du type sphérule d'un produit présentant une forte surfusion tel que le gaïacolate de glycéryle.

Sur le produit obtenu, on observe une répartition granulométrique uniforme.

La figure 2 représente une photographie prise au microscope électronique à balayage (G = 50) qui met en évidence l'aspect de surface des perles obtenues.

La structure originale des produits de l'invention est obtenue grâce à un procédé de fabrication parfaitement adapté.

Le procédé de l'invention de préparation de perles cristallisées d'un produit présentant le phénomène de surfusion consiste donc:
- à faire fondre si nécessaire ledit produit, produit,
- à fragmenter la masse fondue en gouttelettes,
- à refroidir les gouttelettes à une température inférieure à la température de transition vitreuse du produit afin que ces gouttelettes se durcissent par vitrification,
- à maintenir les perles ainsi vitrifiées à une température inférieure à la température de transition vitreuse et à les mettre en contact avec des germes de cristallisation,
- à faire cristalliser les perles en élevant progressivement la température au-dessus de la température de transition vitreuse,
- à récupérer les perles cristallisées.

Dans le procédé de l'invention, on met en oeuvre un produit à l'état fondu.

On peut envisager d'alimenter directement le produit fondu provenant d'une ligne de fabrication. Ceci est particulièrement intéressant lorsque le produit de départ est un produit thermosensible.

Si l'on dispose d'une forme solide pulvérulente, il y a lieu de prévoir une étape de procédé de l'invention qui consiste à faire fondre ledit produit. A cet effet, on chauffe le produit à sa température de fusion. De préférence, on porte le produit à une température légèrement supérieure à sa température de fusion, de préférence supérieure à au plus 5°C par rapport à son point de fusion. Pour le gaïacolate de glycéryle spécifiquement, la température à laquelle est portée celui-ci, est choisie entre 83°C et 88°C.

Cette opération est effectuée, généralement sous agitation.

Dans une étape suivante, on transforme la masse fondue, en gouttelettes. Cette opération peut être réalisée au moyen de tout dispositif de fragmentation, par exemple, par une buse plate à orifice(s) circulaire(s).

Un mode de réalisation préférentielle de l'invention consiste à former les gouttelettes par passage de la masse fondue au travers d'un orifice et tout particulièrement par passage au travers d'une buse.

L'opération suivante est d'assurer le durcissement des gouttelettes en perles vitrifiées par contact avec un fluide froid dont la température est choisie de telle sorte qu'elle soit inférieure à la température de transition vitreuse du produit à mettre en forme.

Par "température de transition vitreuse" on entend, la température à laquelle il y a passage de l'état vitreux à l'état solide non rigide.

La température de transition vitreuse est déterminée d'une manière connue et plus particulièrement selon la norme AFNOR T51-507-2 de décembre 1991.

La température du fluide froid est de préférence, plus basse de 10 à 20°C que la température de transition vitreuse. Dans le cas du gaïacolate de glycéryle, la température du fluide froid est choisie entre -60°C et -30°C, de préférence, entre -60°C et -40°C.

D'une manière générale, la borne inférieure ne présente aucun caractère critique. Elle est uniquement limitée par des considérations technologiques et/ou économiques.

Le fluide froid est plus particulièrement un gaz froid et, de préférence, l'air mais l'invention n'exclut pas un quelconque gaz dans la mesure où il est inerte vis-à-vis du produit à mettre en forme. On peut choisir l'azote mais d'une manière générale, on préfère l'air ou de l'air appauvri en oxygène (par exemple à 10 %) dans le cas où le produit est oxydable ou inflammable.

D'une manière préférentielle, on envoie le courant gazeux froid, à contre-courant du flux de matière.

Le temps de séjour qui est la durée entre la formation de la gouttelette à la sortie de la buse et son arrivée dans le système de récupération se situe avantageusement, entre 1 et 10 secondes et plus préférentiellement entre 1 et 3 secondes.

Une façon d'obtenir le temps de séjour désiré, est de laisser tomber les gouttelettes dans une tour à contre-courant d'un gaz froid tel que précité.

En fin de réaction, on récupère les perles vitrifiées par tout moyen connu, par exemple, par gravité dans un bac de récupération ou, de préférence, selon la technique de lit fluide.

Dans une étape suivante, on réalise la transformation des perles vitrifiées en perles cristallisées.

A cet effet, on maintient les perles vitrifiées à une température inférieure à la température de transition vitreuse, de préférence, inférieure de 5 à 10°C par rapport à la température de transition vitreuse et on les met en contact avec des germes de cristallisation.

Pour des perles de gaïacolate de glycéryle, la température est choisie avantageusement entre -30°C et -35°C.

Les germes de cristallisation sont constitués par une faible quantité de poudre cristallisée de la même nature que le produit à mettre en forme.

La taille des particules de la poudre est très inférieure à celle des perles obtenues, de préférence, le diamètre est inférieur à une valeur représentant le 1/10^{ème} du diamètre des perles obtenues.

On choisit de mettre en oeuvre des germes en une quantité représentant par exemple, de 0,1 à 5 % par rapport au poids des perles vitrifiées.

Afin de déclencher la cristallisation, on remonte progressivement la température à une température au moins égale à la température de transition vitreuse et jusqu'à la température qui correspond à l'exothermie liée à la cristallisation des perles.

La borne supérieure de température se situe de préférence, de 10 à 20°C au-dessus de la température de transition vitreuse.

L'augmentation de température est effectuée de préférence, progressivement. A cet effet, on peut, par exemple, monter la température de 0,5 à 1°C par minute.

La durée de cette opération varie largement et on précise à titre indicatif, qu'une durée de 10 minutes et 2 heures est généralement nécessaire pour obtenir la transformation des perles vitrifiées en perles cristallisées.

On récupère les perles cristallisées par tout moyen connu, mais de préférence, à l'aide d'un dispositif permettant d'évacuer rapidement les calories dégagées par la cristallisation. Ainsi, un lit fluide est bien adapté pour effectuer la dernière étape du procédé de l'invention.

Les perles d'un produit présentant de la surfusion ainsi obtenues selon le procédé de l'invention présentent les caractéristiques explicitées ci-dessus.

En ce qui concerne l'appareillage utilisé pour mettre en oeuvre le procédé de l'invention, il est composé de trois ensembles : un premier ensemble de mise en forme des perles, un deuxième ensemble de récupération des perles vitrifiées et un troisième ensemble de cristallisation des perles vitrifiées.

Le premier ensemble comprend un bac de stockage de préférence agité lorsque le produit à mettre en forme provient d'une ligne de fabrication ou bien un fondoir permettant de fondre le produit s'il est sous forme pulvérulente et une enceinte qui est généralement, une tour d'une hauteur allant de préférence entre 4 et 8 mètres, comprenant dans sa partie supérieure, un dispositif de fragmentation en gouttelettes, de préférence une buse et équipée dans sa partie inférieure d'une ou plusieurs arrivées d'un courant gazeux froid transformant ainsi le bas de la tour en une tour de refroidissement.

Le produit à mettre en forme est introduit par une trémie à double vis, dans un fondoir qui est un réacteur équipé d'un système permettant de réguler la température, par exemple, une double enveloppe, afin de maintenir ledit produit à l'état fondu.

La buse utilisée peut être une buse à un seul trou ou une buse multi-trous avec un nombre de trous qui peut varier entre 1 et 100 trous.

On peut utiliser un système comprenant plusieurs buses, par exemple, 2 buses de préférence amovibles, en parallèle.

Le diamètre des perforations de la buse est fonction de la taille des perles désirées. Il peut être de 50 à 2 000 µm mais il est choisi, de préférence, entre 200 et 600 µm.

La taille de la perforation est toujours inférieure à la taille de la sphérule obtenue. Ainsi, on utilise une buse présentant des perforations d'environ 200 µm pour obtenir des perles présentant un diamètre médian de 550 µm.

La buse utilisée peut être une buse statique mais il est possible de faire appel à une buse soumise à un système de vibration de haute fréquence, par exemple, de 100 à 10 000 hertz.

Le produit fondu arrive dans la buse soit à l'aide d'une pompe volumétrique ou soit par une surpression qui est assurée par un courant gazeux, de préférence, un courant d'azote. La surpression par rapport à la pression atmosphérique est de 5 à 500 %.

La buse est maintenue à une température légèrement supérieure à la température de fusion du produit, de préférence supérieure de 2 à 5°C par rapport à celle-ci.

Au niveau de la buse, il est possible mais non indispensable d'établir un courant gazeux, de préférence un co-courant d'air ou d'azote avec le jet sortant de la buse. Ce courant gazeux a, de préférence, une température comprise entre la température ambiante et 80°C. Le présence de ce co-courant gazeux permet d'obtenir une meilleure régularité de la dimension des perles et évite la coalescence des gouttes.

Dans la partie supérieure de la tour, il peut y avoir sur le paroi interne de la tour, présence de chicanes et de grilles permettant l'homogénéisation du flux gazeux.

Dans le bas de la tour, on introduit un courant de gaz froid, de préférence un courant d'air froid pour assurer le durcissement des gouttelettes en perles vitrifiées. Il a une température choisie de telle sorte qu'elle soit inférieure à la température de transition vitreuse du produit à mettre en forme, de préférence, à une température plus basse de 10 à 20°C que la température de transition vitreuse

Le gaz froid sort, de préférence, de la tour, en dessous de la buse, à une distance représentant avantageusement un dixième de la hauteur totale de la zone de refroidissement.

Le système de récupération des perles, en bas de la tour, est choisi de telle sorte qu'il permette de maintenir les perles vitrifiées à une température inférieure à la température de transition vitreuse du produit. Il peut être constitué d'un bac de récupération réfrigéré mais il est préférable de faire appel à un dispositif permettant d'assurer la fluidisation du lit de particules. Il est constitué par un bac, de préférence cylindrique, comportant dans sa partie inférieure, une grille au travers de laquelle est envoyé un courant gazeux, de préférence l'azote, l'air ou de l'air appauvri en oxygène. Le débit gazeux, qui dépend de la taille des particules, doit être tel qu'il maintient les particules en suspension. On précise, à titre d'exemple, qu'il est de 5 à 30 m³/h pour un diamètre de lit fluide de 80 mm.

Dans cette partie de l'appareil, on poursuit nécessairement le refroidissement car la température du gaz de fluidisation (de préférence, l'air) envoyé est à la même température (± 5°C) que la température du courant gazeux froid envoyé, en bas de tour.

Le dispositif de fluidisation dispose d'une sortie permettant l'évacuation des perles vitrifiées.

Le troisième ensemble correspond à une partie de l'appareillage où est effectuée la cristallisation. Cette opération est conduite dans un dispositif de lit fluide ou tout autre dispositif, par exemple un couloir vibrant, permettant de mettre en contact les perles vitrifiées et les germes de cristallisation et d'assurer une remontée lente en température.

Une première variante consiste à effectuer cette opération dans le même dispositif de fluidisation utilisé dans l'étape précédente. Ainsi, une fois les perles vitrifiées formées, on envoie le gaz de fluidisation à une température inférieure à la température de transition vitreuse du produit, de préférence, inférieure de 5 à 10°C puis on introduit les germes de cristallisation soit par l'intermédiaire de la sortie d'évacuation ou par tout moyen prévu à cet effet.

On fait cristalliser les perles en élevant la température de celles-ci au-dessus de la température de transition vitreuse. On envoie donc le gaz de fluidisation à la température adéquate et l'on contrôle l'augmentation de la température qui résulte alors de la cristallisation en refroidissant si nécessaire. La montée de la température est avantageusement de 0,5 à 1°C par minute.

On augmente la température jusqu'à ce que l'on note l'exothermie liée à la cristallisation des perles.

Le débit du gaz de fluidisation est avantageusement élevé afin d'évacuer les calories libérées. A titre d'exemple, on précise qu'il est de 80 à 200 m³/h, par kg de perles, pour un diamètre de lit fluide de 150 mm.

Ensuite, on évacue les perles cristallisées à l'aide d'un dispositif approprié.

Une autre variante du procédé de l'invention consiste à prévoir un deuxième dispositif de fluidisation mis à la suite du précédent.

Les perles vitrifiées provenant du premier lit fluide sont mélangées avec les germes de cristallisation et tombent dans un deuxième lit fluide dont la température du gaz de fluidisation est au moins égale à la température de transition vitreuse, de préférence, légèrement supérieure à ladite température. Comme mentionné précédemment, on veille à une montée progressive en température.

Une fois les perles cristallisées, elles sont évacuées d'une manière classique.

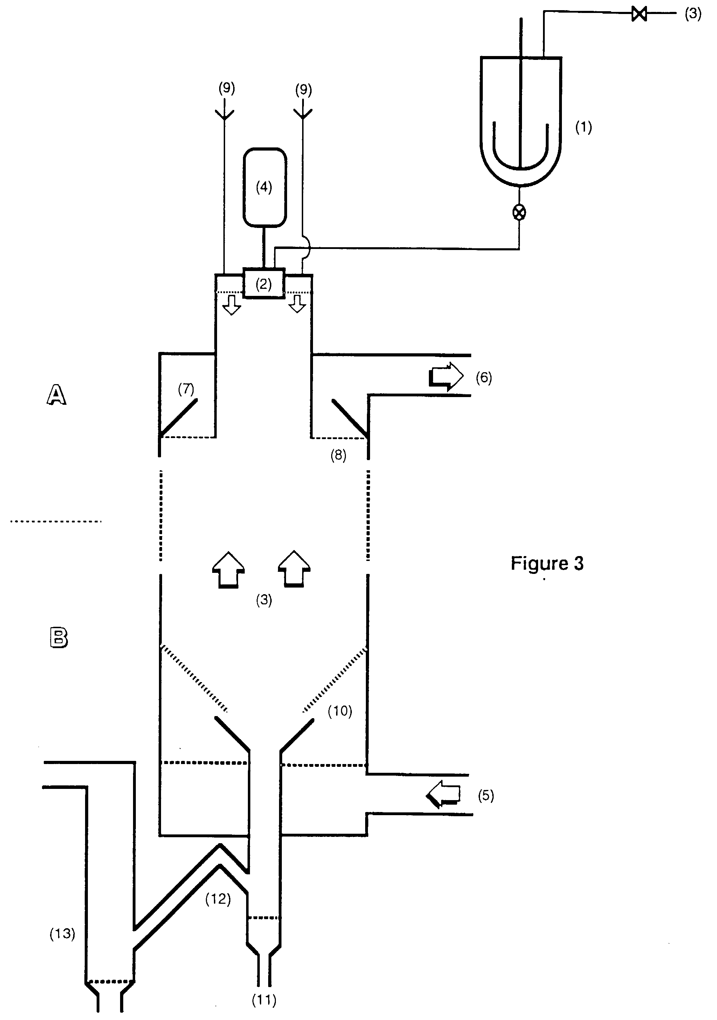

Un mode de réalisation pratique de l'invention est illustré par le dessin annexé sous forme de figure 3.

La figure 3 est une vue latérale schématique d'un appareil adapté à la mise en oeuvre de l'invention.

L'appareil utilisé est constitué de deux parties : la partie supérieure ou tour de prilling (A) et la partie inférieure qui schématise les dispositifs de fluidisation (B).

La poudre à mettre en forme est introduite dans le pot (1) où elle est fondue avant d'être acheminée vers la buse (2). Pour cela, de l'azote (3) est admis en surpression dans le bac à réserve (1).

La tour d'une hauteur de 8 mètres, comprend dans sa partie supérieure une buse (2) solidaire d'un vibrateur (4) et est équipée dans sa partie inférieure d'une arrivée d'un courant d'air froid (5).

L'air de refroidissement introduit en (5) ressort de la tour au point (6) en dessous de la buse (2).

Dans la partie supérieure de la tour, des chicanes (7) ainsi qu'une grille (8) de forme annulaire assurent une distribution homogène du flux gazeux dans la tour.

Un flux d'azote (9) chaud ayant une température comprise entre 20°C et 80°C, de préférence entre 60°C et 80°C, est distribué à co-courant autour de la buse (2).

Dans la partie inférieure de la tour, une grille de forme tronconique (10) permet de collecter les perles vitrifiées solidifiées dans un dispositif de fluidisation comprenant une arrivée d'azote en (11) et une sortie (12) qui sert à transférer les perles vitrifiées vers un second lit fluidisé (13) où sont introduits les germes de cristallisation et où la cristallisation des perles est déclenchée par élévation de leur température au-dessus de la température de transition vitreuse.

On donne ci-après, des exemples de réalisation pratique de l'invention.

### EXEMPLES

On définit, ci-après, le protocole opératoire qui sera repris dans les exemples suivants 1 et 2.

On met en oeuvre 1 kg d'un produit présentant une forte surfusion à savoir le gaïacolate de glycéryle.

Le procédé de l'invention est mis en oeuvre dans l'appareillage décrit ci-dessus et schématisé par la figure 3.

La buse, soumise a des vibrations, présente des caractéristiques précisées dans les exemples suivants.

On introduit la poudre du produit à mettre en forme dans le pot (1).

On fond ledit produit dans le fondoir par chauffage à l'aide d'eau chaude circulant dans la double enveloppe.

La température du produit est de 84°C en (1) pour le gaïacolate de glycéryle. La température en (2), en sortie de buse et le débit du produit sont précisés ci-après.

On introduit en (5) de l'air de refroidissement à un débit de 850 m³/h, soit une vitesse dans la tour de 0,6 m/s. L'air ressort en (6).

Les températures de l'air à l'entrée de la tour (5) et à la sortie de la tour en (6) sont précisées dans un tableau récapitulatif donné dans chaque exemple.

Il en est de même pour la température de l'air de fluidisation en (11).

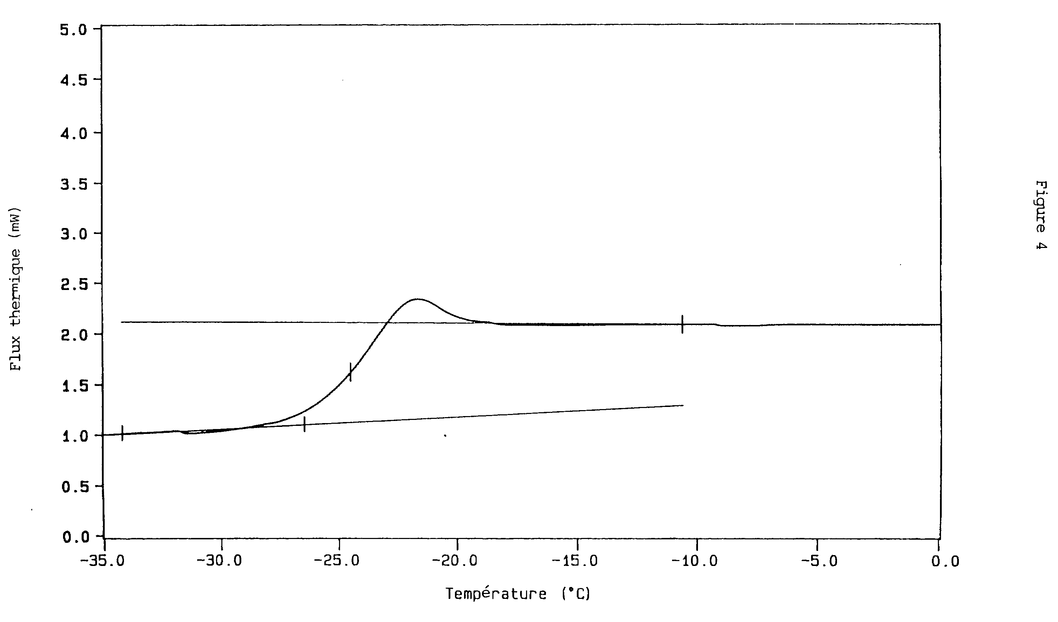

Les perles vitrifiées obtenues sont collectées en (10) et évacuées en (12) vers le lit fluidisé (13) où elles sont maintenues en fluidisation à une température de - 35°C jusqu'à la fin de l'opération de prilling ; la température de transition vitreuse étant située vers -25°C, comme le met en évidence la figure 4 qui est une courbe d'analyse thermique différentielle obtenue pour une vitesse de chauffage de 5°C/min (déterminée sur appareil Perkin-Elmer®) qui représente la variation du flux thermique (exprimé en mW) en fonction de la température (exprimée en °C).

Du gaïacolate de glycéryle en poudre fine est introduit en (13) pour former des germes de cristallisation puis la température de l'air de fluidisation en (13) est élevée à raison de 0,5°C/min.

Lorsque la température atteint 2 à 3°C, on enregistre une exothermie liée à la cristallisation des perles.

Un débit d'air de fluidisation de 100 m³/h en (13) permet d'évacuer rapidement les calories libérées et évite une montée de la température du produit au-delà de 20°C.

### Example 1

Les perles de gaïacolate de glycéryle sont préparées dans un appareillage tel que schématisé par la figure 3, comportant une buse à 1 trou. Le rapport L/D est de 3 ; L représentant la longueur de l'orifice et D le diamètre de l'orifice.

Le procédé mis en oeuvre est tel que décrit ci-dessus : les conditions de marche étant précisées dans le tableau I suivant :

**Tableau I**

| | |
|---|---|
| Diamètre du trou de la buse | 250 µm |
| Fréquence de vibration de la buse (2) | 970 Hz |
| Surpression de l'azote (3) | 0,22 bar |
| Température du produit fondu en (2) | 86°C |
| Débit du produit à la sortie de la buse (2) | 0,410 kg/h |
| Température de l'air à l'entrée de la tour (5) | -60°C |
| Température de l'air à la sortie de la tour (6) | -45°C |
| Température de l'air du lit fluide (11) | -55°C |
| Température de l'air du lit fluide (13) | -35°C |

Après 120 minutes de fonctionnement, on récupère 820 g de perles vitrifiées dans le lit fluide (13).

On ajoute 10 g de poudre de gaïacolate de glycéryle en (13).

On augmente la température de l'air du lit fluide (13).

La vitesse de montée de la température est de 0,5°C/min.

La température finale de fluidisation est de + 18°C.

Après 110 minutes de fluidisation, on récupère 820 g de perles cristallisées ayant un diamètre de 570 µm. Elles sont séparées de l'excès de poudre ayant servi de germes de cristallisation, par simple tamisage.

### Example 2

La préparation des perles de gaïacolate de glycéryle est effectuée comme dans l'exemple 1 : les modifications des paramètres de procédé étant précisées dans le tableau suivant:

**Tableau II**

| | |
|---|---|
| Diamètre du trou de la buse | 400 µm |
| Fréquence de vibration de la buse (2) | 1010 Hz |
| Surpression de l'azote (3) | 0,17 bar |
| Température du produit fondu en (2) | 84°C |
| Débit du produit à la sortie de la buse (2) | 0,870 kg/h |
| Température de l'air à l'entrée de la tour (5) | -60°C |
| Température de l'air à la sortie de la tour (6) | -45°C |
| Température de l'air du lit fluide (11) | -55°C |
| Température de l'air du lit fluide (13) | -35°C |

Après 60 minutes de fonctionnement, on récupère 870 g de perles vitrifiées dans le lit fluide (13).

On ajoute 10 g de poudre de gaïacolate de glycéryle en (13).

On augmente la température de l'air du lit fluide (13).

La vitesse de montée de la température est de 0,5°C/min.

La température finale de fluidisation est de + 18°C.

Après 110 minutes de fluidisation, on récupère 870 g de perles cristallisées ayant un diamètre de 720 µm. Elles sont séparées de l'excès de poudre ayant servi de germes de cristallisation, par simple tamisage.

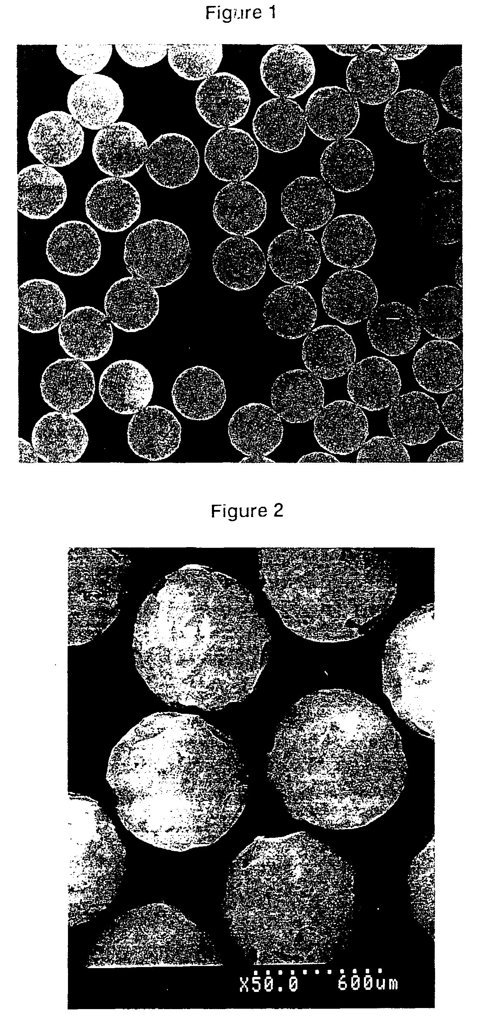

Le produit obtenu présente une morphologie sous forme de perles illustrée par les figures 1 et 2 qui représentent des photographies prises au microscope optique (G = 18) et au microscope à balayage électronique (G = 50).

### Exemple 3

Dans cet exemple, on prépare des perles d'acide 2-(3-benzoylphényl) propionique (Kétoprofène®) dans un appareillage tel que schématisé par la figure 3, comportant une buse à 1 trou avec un rapport LD de 3.

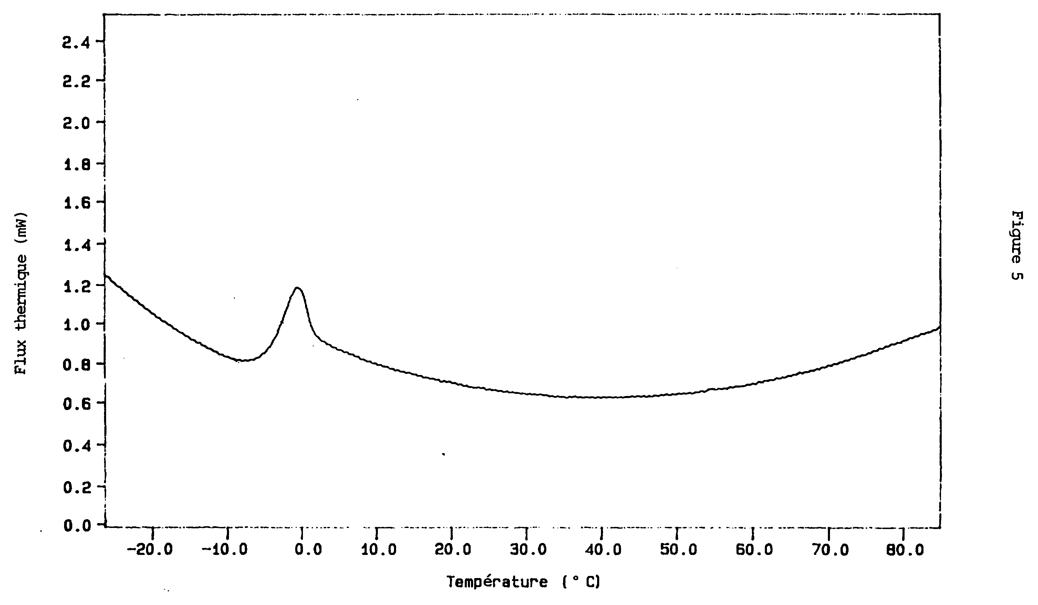

La température de transition vitreuse se situe vers - 5°C comme le met en évidence la figure 5 qui est une courbe d'analyse thermique différentielle obtenue pour une vitesse de chauffage de 5°C/min (déterminée sur appareil Perkin-Elmer®) qui représente la variation du flux thermique (exprimé en mW) en fonction de la température (exprimée en °C).

Les conditions de marche sont précisées dans le tableau III.

**Tableau III**

| | |
|---|---|
| Diamètre du trou de la buse | 400 µm |
| Fréquence de vibration de la buse (2) | 435 Hz |
| Surpression de l'azote (3) | 0,51 bar |
| Température du produit fondu en (2) | 95°C |
| Débit du produit à la sortie de la buse (2) | 0,840 kg/h |
| Température de l'air à l'entrée de la tour (5) | -35°C |
| Température de l'air à la sortie de la tour (6) | -20°C |
| Température de l'air du lit fluide (11) | -35°C |
| Température de l'air du lit fluide (13) | -10°C |

Après 60 minutes de fonctionnement, on récupère 840 g de perles vitrifiées dans le lit fluide (13).

On ajoute 10 g de poudre d'acide 2-(3-benzoylphényl)propionique en (13).

On augmente la température de l'air du lit fluide (13).

La vitesse de montée de la température est de 0,5°C/min.

La température finale de fluidisation est de 40°C.

Après 100 minutes de fluidisation, on récupère 840 g de perles cristallisées ayant un diamètre de 940 µm. Elles sont séparées de l'excès de poudre ayant servi de germes de cristallisation, par simple tamisage.

## Revendications

1. Procédé de préparation de perles cristallisées d'un produit présentant de la surfusion caractérisé par le fait qu'il consiste :
- à faire fondre si nécessaire ledit produit,
- à fragmenter la masse fondue en gouttelettes,
- à refroidir les gouttelettes à une température inférieure à la température de transition vitreuse du produit afin que ces gouttelettes se durcissent par vitrification,
- à maintenir les perles ainsi vitrifiées à une température inférieure à la température de transition vitreuse et à les mettre en contact avec des germes de cristallisation,
- à faire cristalliser les perles en élevant la température au-dessus de la température de transition vitreuse,
- à récupérer les perles cristallisées.

2. Procédé selon la revendication 1 caractérisé par le fait qu'il consiste à faire passer la masse fondue dans une buse de façon à former des gouttelettes, à solidifier ces dernières en les laissant tomber dans une tour à contre-courant d'un gaz froid puis à récupérer les perles vitrifiées obtenues.

3. Procédé selon l'une des revendications 1 et 2 caractérisé par le fait que le produit à mettre en forme est le gaïacolate de glycéryle, l'acide 2-(3-benzoylphényl)propionique, l'acide 2-(4-isobutylphényl)propionique, le dicarbonate de 2-méthyl-2-propyl-1,3-propanediol.

4. Procédé selon l'une des revendications 1 à 3 caractérisé par le fait que l'on fait fondre le produit à mettre en forme à sa température de fusion, de préférence à une température supérieure à au plus 5°C par rapport à son point de fusion.

5. Procédé selon la revendication 4 caractérisé par le fait que le gaïacolate de glycéryle est fondu à une température comprise entre 83°C et 88°C.

6. Procédé selon la revendication 2 caractérisé par le fait que la buse utilisée est une buse à un seul trou ou une buse multi-trous avec un nombre de trous variant entre 1 et 100 trous.

7. Procédé selon la revendication 6 caractérisé par le fait que la buse utilisée comporte des perforations dont le diamètre varie entre 50 à 2 000 µm et se situe, de préférence, entre 200 et 600 µm.

8. Procédé selon l'une des revendications 6 et 7 caractérisé par le fait que la buse utilisée est une buse statique mais de préférence une buse soumise à un système de vibration de haute fréquence, de préférence, de 100 à 10 000 hertz.

9. Procédé selon l'une des revendications 1 à 8 caractérisé par le fait que les gouttelettes sont mises en contact avec un fluide froid, de préférence un gaz froid et plus particulièrement l'azote, l'air ou de l'air appauvri en oxygène, dont la température est inférieure à la température de transition vitreuse du produit à mettre en forme, de préférence, à une température plus basse de 10 à 20°C que la température de transition vitreuse.

10. Procédé selon la revendication 9 caractérisé par le fait que la température du gaz froid est choisie entre -60°C et -30°C, de préférence, entre -60°C et -40°C, pour la mise en forme du gaïacolate de glycéryle.

11. Procédé selon l'une des revendications 1 à 10 caractérisé par le fait que l'on envoie un co-courant gazeux, de préférence un co-courant d'air ou d'azote avec le jet sortant de la buse à une température comprise de préférence, entre la température ambiante et 80°C.

12. Procédé selon l'une des revendications 1 à 11 caractérisé par le fait que le temps de séjour de la gouttelette à la sortie de la buse et son arrivée dans le système de récupération se situe avantageusement, entre 1 et 10 secondes et plus préférentiellement entre 1 et 3 secondes.

13. Procédé selon l'une des revendications 1 à 12 caractérisé par le fait que l'on récupère les perles vitrifiées par tout moyen connu, de préférence selon la technique de lit fluide.

14. Procédé selon l'une des revendications 1 à 13 caractérisé par le fait que l'on maintient les perles vitrifiées à une température inférieure à la température de transition vitreuse, de préférence, inférieure de 5 à 10°C par rapport à la température de transition vitreuse et on les met en contact avec des germes de cristallisation.

15. Procédé selon la revendication 14 caractérisé par le fait que, la température est choisie entre -30°C et -35°C, pour des perles de gaïacolate de glycéryle.

16. Procédé selon l'une des revendications 1 à 15 caractérisé par le fait que les germes de cristallisation sont constitués par une faible quantité de poudre cristallisée de la même nature que le produit à mettre en forme.

17. Procédé selon l'une des revendications 1 à 16 caractérisé par le fait que la quantité de germes à mettre en oeuvre représente de 0,1 à 5 % par rapport au poids des perles vitrifiées.

18. Procédé selon l'une des revendications 14 à 17 caractérisé par le fait que l'on monte progressivement la température à une température au moins égale à la température de transition vitreuse, de préférence, de 10 à 20°C au-dessus de la température de transition vitreuse.

19. Procédé selon la revendication 18 caractérisé par le fait que la température est augmentée progressivement de préférence, de 0,5 à 1°C par minute.

20. Procédé selon l'une des revendications 1 à 19 caractérisé par le fait que l'on récupère les perles cristallisées, de préférence selon la technique de lit fluide.

21. Perles cristallisées d'un produit présentant une surfusion obtenues selon le procédé décrit dans l'une des revendications 1 à 20 et exemptes d'additifs de cristallisation.

22. Perles cristallisées d'un produit présentant une surfusion telle que l'on mette en évidence une transition vitreuse et ne comprenant pas d'additifs de cristallisation.

23. Perles cristallisées selon l'une des revendications 21 et 22 caractérisées par le fait qu'elles ont une taille exprimée par le diamètre médian (d₅₀) variant entre 100 µm et 3000 µm ; de préférence, entre 500 µm à 2 000 µm.

24. Perles cristallisées selon la revendication 21 à 23 caractérisées par le fait que le produit mis en forme est le gaïacolate de glycéryle.

25. Perles cristallisées selon la revendication 24 caractérisées par le fait qu'elles ont une taille exprimée par le diamètre médian (d₅₀) variant de 500 µm à 1 000 µm.

26. Perles selon l'une des revendications 21 à 23 caractérisées par le fait que le produit mis en forme est l'acide 2-(3-benzoylphényl)propionique, l'acide 2-(4-isobutylphényl)propionique, le dicarbonate de 2-méthyl-2-propyl-1,3-propanediol.

27. Perles cristallisées exemptes d'additifs de cristallisation et comprenant au moins du gaïacolate de glycéryle.

28. Appareillage composé de trois ensembles comprenant un premier ensemble de mise en forme des perles, un deuxième ensemble de récupération des perles vitrifiées et un troisième ensemble de cristallisation des perles vitrifiées.

29. Appareillage selon la revendication 28 caractérisé par le fait que le premier ensemble comprend une tour comprenant dans sa partie supérieure, un dispositif de fragmentation en gouttelettes, de préférence une buse et équipée dans sa partie inférieure d'une ou plusieurs arrivées d'un courant gazeux froid.

30. Appareillage selon la revendication 28 caractérisé par le fait que le deuxième ensemble comprend un dispositif permettant d'assurer un premier lit fluidisé.

31. Appareillage selon la revendication 28 caractérisé par le fait que le troisième ensemble comprend un dispositif permettant d'assurer un deuxième lit fluidisé.

## Patentansprüche

1. Verfahren zur Herstellung von kristallisierten Perlen aus einem unterkühlten Produkt, dadurch gekennzeichnet, daß es darin besteht:
- falls erforderlich das genannte Produkt zu schmelzen,
- die Schmelze in Tröpfchen zu zerteilen,
- die Tröpfchen bei einer Temperatur abzukühlen, die unter der Glasübergangstemperatur des Produktes liegt, damit diese Tröpfchen durch Verglasung aushärten,
- die so verglasten Perlen bei einer Temperatur unterhalb der Glasübergangstemperatur zu halten und sie mit Kristallisationskeimen zusammenzubringen,
- die Perlen durch Erhöhen der Temperatur über die Glasübergangstemperatur kristallisieren zu lassen,
- die kristallisierten Perlen aufzufangen.

2. Verfahren nach Anspruch 1, dadurch gekennzeichnet, daß es darin besteht, die Schmelze durch eine Düse zu leiten, so daß Tröpfchen geformt werden, letztere erstarren zu lassen, indem man sie in einem Turm mit einem Gegenstrom eines kalten Gases fallen läßt, und schließlich die erhaltenen verglasten Perlen aufzufangen.

3. Verfahren nach einem der Ansprüche 1 und 2, dadurch gekennzeichnet, daß das zu formende Produkt Glycerylguajakolat, 2-(3-Benzoylphenyl)propionsäure, 2-(4-Isobutylphenyl)propionsäure, das Dicarbonat von 2-Methyl-2-propyl-1,3-propandiol ist.

4. Verfahren nach einem der Ansprüche 1 bis 3, dadurch gekennzeichnet, daß man das zu formende Produkt bei seiner Schmelztemperatur zum Schmelzen bringt, vorzugsweise bei einer Temperatur, die höchstens 5 °C höher als sein Schmelzpunkt ist.

5. Verfahren nach Anspruch 4, dadurch gekennzeichnet, daß das Glycerylguajakolat bei einer Temperatur zwischen 83 °C und 88 °C geschmolzen ist.

6. Verfahren nach Anspruch 2, dadurch gekennzeichnet, daß die verwendete Düse eine Düse mit einer Öffnung oder eine Düse mit mehreren Öffnungen ist, wobei die Anzahl der Öffnungen zwischen 1 und 100 schwankt.

7. Verfahren nach Anspruch 6, dadurch gekennzeichnet, daß die verwendete Düse Perforationen enthält, deren Durchmesser zwischen 50 und 2.000 µm schwankt und vorzugsweise zwischen 200 und 600 µm liegt.

8. Verfahren nach einem der Ansprüche 6 und 7, dadurch gekennzeichnet, daß die verwendete Düse eine statische Düse ist, vorzugsweise aber eine Düse, die mit einem Vibrationssystem einer hohen Frequenz, vorzugsweise zwischen 100 und 10.000 Hertz, verbunden ist.

9. Verfahren nach einem der Ansprüche 1 bis 8, dadurch gekennzeichnet, daß die Tröpfchen mit einem kalten Fluid, vorzugsweise mit einem kalten Gas und noch spezieller mit Stickstoff, Luft oder sauerstoffarmer Luft zusammengebracht werden, deren Temperatur unter der Glasübergangstemperatur des zu formenden Produktes liegt, vorzugsweise bei einer Temperatur, die 10 bis 20 °C unter der Glasübergangstemperatur liegt.

10. Verfahren nach Anspruch 9, dadurch gekennzeichnet, daß die Temperatur des kalten Gases zwischen -60 °C und -30 °C, vorzugsweise zwischen -60 °C und -40 °C, für das Formen des Glycerylguajakolats gewählt ist.

11. Verfahren nach einem der Ansprüche 1 bis 10, dadurch gekennzeichnet, daß man einen gasförmigen Gleichstrom einleitet, vorzugsweise einen Gleichstrom aus Luft oder Stickstoff, wobei der aus der Düse kommende Strahl eine Temperatur hat, die vorzugsweise zwischen Umgebungstemperatur und 80 °C liegt.

12. Verfahren nach einem der Ansprüche 1 bis 11, dadurch gekennzeichnet, daß die Verweilzeit des Tröpfchens vom Düsenausgang bis zu seiner Ankunft in der Auffangvorrichtung vorteilhafterweise zwischen 1 und 10 Sekunden und noch bevorzugter zwischen 1 und 3 Sekunden liegt.

13. Verfahren nach einem der Ansprüche 1 bis 12, dadurch gekennzeichnet, daß man die verglasten Perlen mit allen bekannten Mitteln, vorzugsweise mit der Fließbettechnik, auffängt.

14. Verfahren nach einem der Ansprüche 1 bis 13, dadurch gekennzeichnet, daß man die verglasten Perlen bei einer Temperatur unterhalb der Glasübergangstemperatur hält, die vorzugsweise 5 bis 10 °C unter der Glasübergangstemperatur liegt, und sie mit Kristallisationskeimen zusammenbringt.

15. Verfahren nach Anspruch 14, dadurch gekennzeichnet, daß die Temperatur für Perlen aus Glycerylguajakolat zwischen -30 °C und -35 °C gewählt ist.

16. Verfahren nach einem der Ansprüche 1 bis 15, dadurch gekennzeichnet, daß die Kristallisationskeime aus einer geringen Menge eines kristallisierten Pulvers derselben Art wie das zu formende Produkt bestehen.

17. Verfahren nach einem der Ansprüche 1 bis 16, dadurch gekennzeichnet, daß die Menge der eingesetzten Keime 0,1 bis 5 % des Gewichts der verglasten Perlen entspricht.

18. Verfahren nach einem der Ansprüche 14 bis 17, dadurch gekennzeichnet, daß man die Temperatur progressiv erhöht bis zu einer Temperatur, die mindestens gleich der Glasübergangstemperatur ist und vorzugsweise 10 bis 20 °C über der Glasübergangstemperatur liegt.

19. Verfahren nach Anspruch 18, dadurch gekennzeichnet, daß die Temperatur vorzugsweise 0,5 bis 1 °C pro Minute progressiv erhöht wird.

20. Verfahren nach einem der Ansprüche 1 bis 19, dadurch gekennzeichnet, daß man die kristallisierten Perlen auffängt, vorzugsweise mit der Fließbetttechnik.

21. Kristallisierte Perlen aus einem unterkühlten Produkt, die nach einem Verfahren erhalten worden sind, das in einem der Ansprüche 1 bis 20 beschrieben ist und die frei von Kristallisationszusätzen sind.

22. Kristallisierte Perlen aus einem unterkühlten Produkt, bei denen ein Glasübergang deutlich erkennbar ist und die keine Kristallisationszusätze enthalten.

23. Kristallisierte Perlen nach einem der Ansprüche 21 und 22, dadurch gekennzeichnet, daß sie eine als mittlerer Durchmesser (d₅₀) definierte Größe aufweisen, die zwischen 100 µm und 3.000 µm, vorzugsweise zwischen 500 um und 2.000 um, schwankt.

24. Kristallisierte Perlen nach einem der Ansprüche 21 bis 23, dadurch gekennzeichnet, daß das geformte Produkt Glycerylguajakolat ist.

25. Kristallisierte Perlen nach Anspruch 24, dadurch gekennzeichnet, daß ihre als mittlerer Durchmesser (d₅₀) definierte Größe zwischen 500 µm und 1.000 µm schwankt.

26. Perlen nach einem der Ansprüche 21 bis 23, dadurch gekennzeichnet, daß das geformte Produkt 2-(3-Benzoylphenyl)propionsäure, 2-(4-Isobutylphenyl)-propionsäure, das Dicarbonat von 2-Methyl-2-propyl-1,3-propandiol ist.

27. Kristallisierte Perlen, die frei von Kristallisationszusätzen sind und mindestens Glycerylguajakolat enthalten.

28. Vorrichtung, die sich aus drei Anordnungen zusammensetzt, enthaltend eine erste Anordnung zum Formen der Perlen, eine zweite Anordnung zum Auffangen der verglasten Perlen und eine dritte Anordnung zur Kristallisation der verglasten Perlen.

29. Vorrichtung nach Anspruch 28, dadurch gekennzeichnet, daß die erste Anordnung einen Turm enthält, der in seinem oberen Teil eine Vorrichtung zum Zerteilen in Tröpfchen, vorzugsweise eine Düse und in seinem unteren Teil eine oder mehrere Zuführungen eines kalten Gasstroms enthält.

30. Vorrichtung nach Anspruch 28, dadurch gekennzeichnet, daß die zweite Anordnung eine Vorrichtung enthält, mit der ein erstes Fließbett erzeugt werden kann.

31. Vorrichtung nach Anspruch 28, dadurch gekennzeichnet, daß die dritte Anordnung eine Vorrichtung enthält, mit der ein zweites Fließbett erzeugt werden kann.

## Claims

1. Process for the preparation of crystallized pearls of a product exhibiting supercooling, characterized in that it consists:
- in melting, if necessary, the said product,
- in breaking up the molten mass into droplets,
- in cooling the droplets to a temperature below the glass transition temperature of the product, so that these droplets harden by vitrification,
- in maintaining the pearls thus vitrified at a temperature below the glass transition temperature and in placing them in contact with crystallization seeds,
- in crystallizing the pearls by raising the temperature above the glass transition temperature,
- in recovering the crystallized pearls.

2. Process according to Claim 1, characterized in that it consists in passing the molten mass through a nozzle so as to form droplets, in solidifying these droplets and in allowing them to fall in a tower with a countercurrent of a cold gas, and then in recovering the vitrified pearls obtained.

3. Process according to either of Claims 1 and 2, characterized in that the product to be shaped is glycerylguaiacolate, 2-(3-benzoylphenyl)propionic acid, 2-(4-isobutylphenyl)propionic acid or 2-methyl-2-propyl-1,3-propanediol dicarbonate.

4. Process according to one of Claims 1 to 3, characterized in that the product to be shaped is melted at its melting point, preferably at a temperature not more than 5°C above its melting point.

5. Process according to Claim 4, characterized in that the glyceryl guaiacolate is melted at a temperature of between 83°C and 88°C.

6. Process according to Claim 2, characterized in that the nozzle used is a one-hole nozzle or a multi-hole nozzle with a number of holes ranging between 1 and 100 holes.

7. Process according to Claim 6, characterized in that the nozzle used contains perforations whose diameter ranges between 50 and 2,000 µm and is preferably between 200 and 600 µm.

8. Process according to either of Claims 6 and 7, characterized in that the nozzle used is a static nozzle but preferably a nozzle subjected to a system of high frequency vibration, preferably at 100 to 10,000 hertz.

9. Process according to one of Claims 1 to 8, characterized in that the droplets are placed in contact with a cold fluid, preferably a cold gas and more particularly nitrogen, air or oxygen-depleted air, whose temperature is below the glass transition temperature of the product to be shaped, preferably at a temperature 10 to 20°C lower than the glass transition temperature.

10. Process according to Claim 9, characterized in that the temperature of the cold gas is chosen between -60°C and -30°C, preferably between -60°C and-40°C, in order to shape the glyceryl guaiacolate.

11. Process according to one of Claims 1 to 10, characterized in that a gas co-current, preferably a co-current of air or of nitrogen, is sent with the jet leaving the nozzle at a temperature which is preferably between room temperature and 80°C.

12. Process according to one of Claims 1 to 11, characterized in that the residence time of the droplet from leaving the nozzle to its arrival in the recovery system is advantageously between 1 and 10 seconds and more preferably between 1 and 3 seconds.

13. Process according to one of Claims 1 to 12, characterized in that the vitrified pearls are recovered by any known means, preferably according to the fluid bed technique.

14. Process according to one of Claims 1 to 13, characterized in that the vitrified pearls are maintained at a temperature below the glass transition temperature, preferably 5 to 10°C below the glass transition temperature, and are placed in contact with crystallization seeds.

15. Process according to Claim 14, characterized in that the temperature is chosen between -30°C and-35°C for glyceryl guaiacolate pearls.

16. Process according to one of Claims 1 to 15, characterized in that the crystallization seeds consist of a small amount of crystalline powder of the same nature as the product to be shaped.

17. Process according to one of Claims 1 to 16, characterized in that the amount of seeds to be used represents from 0.1 to 5% relative to the weight of the vitrified pearls.

18. Process according to one of Claims 14 to 17, characterized in that the temperature is gradually raised to a temperature at least equal to the glass transition temperature, preferably 10 to 20°C above the glass transition temperature.

19. Process according to Claim 18, characterized in that the temperature is gradually raised, preferably by 0.5 to 1°C per minute.

20. Process according to one of Claims 1 to 19, characterized in that the crystallized pearls are recovered, preferably according to the fluid bed technique.

21. Crystallized pearls of a product exhibiting supercooling, these pearls being obtained according to the process described in one of Claims 1 to 20, and examples of crystallization additives.

22. Crystallized pearls of a product exhibiting supercooling such that a glass transition is observed, and which comprise no crystallization additives.

23. Crystallized pearls according to either of Claims 21 and 22, characterized in that they have a size, expressed by the median diameter (d₅₀), ranging between 100 µm and 3,000 µm; preferably between 500 µm and 2,000 µm.

24. Crystallized pearls according to Claims 21 to 23, characterized in that the product shaped is glyceryl guaiacolate.

25. Crystallized pearls according to Claim 24, characterized in that they have a size, expressed by the median diameter (d₅₀), ranging from 500 µm to 1,000 µm.

26. Pearls according to one of Claims 21 to 23, characterized in that the product shaped is 2-(3-benzoylphenyl)propionic acid, 2-(4-isobutylphenyl)-propionic acid or 2-methyl-2-propyl-1,3-propanediol dicarbonate.

27. Crystallized pearls free of crystallization additives and comprising at least glyceryl guaiacolate.

28. Apparatus composed of three assemblies comprising a first assembly for shaping the pearls, a second assembly for recovery of the vitrified pearls and a third assembly for crystallization of the vitrified pearls.

29. Apparatus according to Claim 28, characterized in that the first assembly comprises a tower comprising, in its upper part, a device for fragmentation into droplets, preferably a nozzle, and equipped in its lower part with one or more cold gas stream inlets.

30. Apparatus according to Claim 28, characterized in that the second assembly comprises a device which makes it possible to provide a first fluidized bed.

31. Apparatus according to Claim 28, characterized in that the third assembly comprises a device which makes it possible to provide a second fluidized bed.
